# EUROPÄISCHE PATENTSCHRIFT

(11) **EP 0 310 014 B1**
(45) Veröffentlichungstag und Bekanntmachung des Hinweises auf die Patenterteilung: **21.06.1995**
(21) Anmeldenummer: 88115957.8
(22) Anmeldetag: 28.09.1988
(51) Int. Cl.: C12Q 1/34, C09B 43/18

(54) **Chromogene Substrate**
Chromogene substrate
Substrat chromogène

(30) Priorität: 30.09.1987 DE 3732871
(43) Veröffentlichungstag der Anmeldung: 05.04.1989
(73) Patentinhaber: BEHRINGWERKE Aktiengesellschaft, 35001 Marburg (DE)
(72) Erfinder: Habenstein, Klaus, Dr., D-3552 Wetter (DE)

(56) Entgegenhaltungen:
- EP-A- 0 008 428
- EP-A- 0 014 929
- EP-A- 0 061 731
- CH-A- 333 924
- DE-A- 2 841 380

## Beschreibung

Die Erfindung bezieht sich auf chromogene Substrate für den Nachweis hydrolysierender Enzyme, Verfahren zum Herstellen dieser chromogenen Substrate und die Verwendung der chromogenen Substrate.

Hydrolysierende Enzyme, die sogenannten Hydrolasen, sind im tierischen Organismus für eine Vielzahl von Reaktionen verantwortlich. Dabei werden Hydrolasen verschiedener Funktion nach ihrer Spezifität unterschieden:
1. Esterasen, wie z.B. die Acetylcholinesterase, die Carbonylester hydrolysieren,
2. Glykosidasen, wie z.B. die ß-D-Galactosidase, die die O-glykosidische Verknüpfung von Zucker untereinander oder mit Alkoholen hydrolysieren,
3. Phosphatasen, wie z.B. alkalische Phosphatase, die Phosphorsäureester hydrolysieren und
4. Sulfatasen, wie z.B. Iduronatsulfatase, die Schwefelsäureester hydrolysieren.

Die Hydrolasen gehören zu den wichtigsten Enzymen des tierischen Organismus. Ihr Fehlen, vermindertes oder vermehrtes Vorkommen deutet häufig auf ernste Erkrankungen des Organismus hin. Ein bekanntes Beispiel ist die Mucopolysaccharidose; diese rezessiv vererbliche Krankheit, für die 7 verschiedene Manifestationsformen unterscheidbar sind, beruht auf einem genetisch determinierten Defekt von Hydrolasen, z.B. der ß-Galaktosidase im Fall der Morbus Morquio und der Iduronatsulfatsulfatase bei Morbus Hunter. Morbus Morquio beispielsweise kann durch Bestimmung des ß-D-Galactosidase-Spiegels von Fibroblasten oder Leucozyten eindeutig diagnostiziert werden. Der Spiegel einer anderen Glykosidase, der Amylase, in Blut oder Harn wird zur Diagnose von Pankreaserkrankungen herangezogen. Darüber hinaus finden hydrolytische Enzyme als diagnostische Hilfsmittel, sogenannte Marker, z.B. in Enzymimmunoassays Verwendung.

Die Quantifizierung hydrolysierender Enzyme zu diagnostischen Zwecken setzt hochempfindliche und spezifische Nachweissysteme voraus, um selbst geringe Enzymkonzentrationen exakt bestimmen zu können. Dabei sind die natürlichen Substrate zum Nachweis ungeeignet, da schon vor der Durchführung des Tests Hydrolyseprodukte in den Proben vorhanden sind oder aber die Hydrolyseprodukte sehr schwer bestimmbar sind. Im Stand der Technik werden daher künstliche Substrate verwendet, deren Hydrolyseprodukte physikalisch oder chemisch nachweisbar sind. In der Regel erfolgt der Nachweis durch Bestimmen der in der hydrolytischen Reaktion freigesetzten Mengen an fluoreszierenden oder stark absorbierenden chromogenen Substanzen. Ein nahezu universell einsetzbares und daher vielfach genutztes Chromogensystem ist das der Nitrophenole. Allerdings ist deren sehr pH-abhängige gelbe Farbentwicklung selbst für photometrische Auswertungen nicht problemlos und für visuelle Auswertungen ungeeignet. Fluorogene Nachweissysteme, z. B. Fluoreszeine, oder Methylumbelliferone können überhaupt nicht visuell, sondern nur mit Instrumenten nachgewiesen werden, andere chromogene Substrate, z.B. Phenoxazine, wie sie in der EP 156 347 beschrieben sind, und Phenothiazine, entsprechend der EP 157 384, weisen bereits im gebundenen Zustand eine erhebliche Eigenabsorption auf. Wieder andere Substrate, z.B. Phenol- und Naphtholderivate und auch Indoxyl bedürfen nach der hydrolytischen Reaktion einer weiteren chemischen Reaktion, um die chromogene Molekülgruppe zur farbigen Verbindung umzusetzten. Viele chromogene Substrate sind darüberhinaus nur im alkalischen pH-Bereich zur Bestimmung hydrolysierender Enzyme einsetzbar.

In der EP-A3 -0 061 731 werden Phosphorsäureester beschrieben, die als Substrate zur quantitativen Bestimmung der alkalischen Phospatase geeignet sind.

In der Patentschrift CH 333 924 wird ein Verfahren zur Herstellung von Azofarbstoffen beschrieben, das auch eine Sulfatierung von Azofarbstoffen betrifft.

Der vorliegenden Erfindung lag daher die Aufgabe zugrunde, chromogene Substrate für den Nachweis hydrolysierender Enzyme zur Verfügung zu stellen, deren Hydrolyse unabhängig vom pH-Bereich hochempfindlich und spezifisch zu meßbaren Signalen führt.

Diese Aufgabe wird erfindungsgemäß dadurch gelöst, daß das chromogene Substrat eine Azofarbstoffverbindung mit der allgemeinen Formel ist:

A - N = N - B (OR)

wobei A) einen zyklischen 5- oder 6gliedrigen, gegebenenfalls benzoanelierten Rest mit bis zu 3 Heteroatomen aus der Gruppe N, S, O bedeutet, der gegebenenfalls durch Halogen-, Nitro-, Alkyl-, Alkoxy- oder Sulfonatgruppen substituiert sein kann, B) Piridinyl bedeutet, das gegebenenfalls durch Halogen-, Alkyl-, Alkoxy-, Dialkylamino- oder Morpholinoreste substituiert sein kann.
und R ein durch enzymatische Hydrolyse freisetzbarer Rest ist, ausgenommen ein Carbonylrest.

Eine besonders bevorzugte Ausführungsform sieht die Verwendung von modifizierten oder unmodifizierten Zuckerresten oder von Phosphatresten oder von Sulfatresten als Rest R vor.

Überraschenderweise hat es sich gezeigt, daß die erfindungsgemäßen Azofarbstoffverbindungen in Abhängigkeit vom gewählten Rest R mit Hydrolasen verschiedener Spezifität in Reaktion treten, sowohl visuell als auch photometrisch erfaßbare Farbwechsel im alkalischen wie im sauren pH-Bereich zeigen und der Farbwechsel im sauren pH-Bereich durch Komplexierung deutlich verstärkt werden kann.

Aus der deutschen Offenlegungsschrift 28 36 644 ist zwar die Verwendung von chromogenen Azofarbstoffverbindungen der genannten allgemeinen Formel für den Nachweis von Leucozytenesterasen schon bekannt, doch handelt es sich hierbei um Substrate ausschließlich für Carbonylesterasen, in denen der Rest R einen Carbonsäurerest oder einen mit einer in der Peptidchemie üblichen Stickstoffschutzgruppe versehenen Aminosäure- oder Peptidrest bedeutet. Für diese Azofarbstofverbindungen ist darüber hinaus nur die Verwendung im alkalischen pH-Bereich beschrieben.

Durch geeignete Wahl der Gruppen A und B in der allgemeinen Formel

A - N = N - B (OR)

lassen sich maßgeschneiderte Substrate für die speziellen analytischen Probleme herstellen. Dabei kann A) ein zyklischer 5- oder 6-gliedriger, gegebenenfalls benzoanelierter Rest mit bis zu 3 Heteroatomen aus der Gruppe N, S, O sein, der gegebenenfalls durch Halogen-, Nitro-, Alkyl-, Alkoxy- oder Sulfonatgruppen substituiert sein kann, während B) ein Piridinyl, sein kann, das gegebenenfalls durch Halogen-, Alkyl-, Alkoxy-, Dialkylamino- oder Morpholinoreste substituiert sein kann. Der Halogenrest kann dabei ein Chlor-, Brom- oder Jodrest sein, bevorzugt ein Chlor- oder Bromrest; die Methyl- bzw. Alkoxygruppen umfassen Ketten mit 1-6 C-Atomen, bevorzugt mit 1-3 C-Atomen. In Abhängigkeit von der Wahl der Gruppen A und B weisen die freien Chromogene eine hohe Farbintensität, d.h. einen hohen molaren Extinktionskoeffizienten auf, der somit hochempfindliche Enzymassays ermöglicht. Besonders bevorzugt sind dabei Azofarbstoffe, deren Verbindungen, also die gebundenen Chromogene, die die chromogenen Substrate darstellen, möglichst gering gefärbt sind, während das freie Chromogen, also der aus der Azofarbstoffverbindung durch Hydrolyse freigesetzte Azofarbstoff, sehr stark gefärbt ist. Beispielsweise lassen sich farblose bis cremefarbene Galaktoside herstellen, die durch Hydrolyse zu einem rotvioletten bis blauen freien Chromogen umgesetzt werden. Bei bevorzugten erfindungsgemäßen chromogenen Substraten ist darüberhinaus die Überlappung der Absorptionsspektren von gebundenem Chromogen und freiem Chromogen so gering wie möglich. Die Löslichkeit des chromogenen Substrates ist ein weiterer, stark von der Wahl der Gruppen A und B abhängiger Parameter, an den je nach Verwendungszweck des chromogenen Substrates höchst unterschiedliche Anforderungen gestellt werden. So wird eine gute Löslichkeit von chromogenem Substrat und freiem Chromogen in wässrigen Medien benötigt, falls die Quantifizierung der Reaktion photometrisch erfolgen soll, während trägergebundene chromogene Substrate, z.B.nach Fixierung auf Papier oder Filmen, zur Verminderung von Ausblutung in wässriger Lösung Chromogene mit geringer Löslichkeit in wässrigen Medien voraussetzen.

Besonders bevorzugt sind chromogene Substrate, deren freies Chromogen nach Hydrolyse durch Komplexierung mit geeigneten Metallionen ein bathochrom verschiebbares Absorptionsmaximum aufweist. Bevorzugte Metallionen für die Komplexbildung sind Kupfer-, Kobalt-, Nickel- und Zinkionen.

Besonders geeignete chromogene Substrate sind Substrate der oben angegebenen allgemeinen Formel, in denen
A - : die Struktur 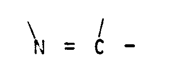 enthält, wobei das gezeigte Kohlenstoffatom der an die Diazogruppe gebundene Bestandteil von A ist
   und
- B (OR) : die Struktur 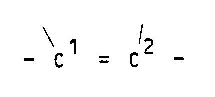 enthält, wobei das gezeigte Kohlenstoffatom C¹ der an die Diazogruppe gebundene Bestandteil von B ist,
wobei A, B und R die in den Ansprüchen 1 und/oder 2 angegebene Bedeutung haben. Diese Substrate weisen durch die komplexbedingte Absorptionsänderung einen über den durch Hydrolyse des chromogenen Substrates erreichbaren hinausgehenden Effekt auf. Bemerkenswerterweise tritt dieser Effekt sowohl im sauren als auch im alkalischen pH-Bereich auf.

Besonders bevorzugte Azofarbstoffe sind
Thiazol-2-azo-2′-pyridin-3′-ol
4,5-Dimethylthiazol-2-azo-2′-pyridin-3′-ol
6-Ethoxybenzthiazol-2-azo-2′-pyridin-3′-ol
Benzthiazol-2-azo-2′-pyridin-3′-ol
5-Bromthiazol-2-azo-2′-pyridin-3′-ol
Thiazol-2-azo-6′-(2′-brom-3′-hydroxypyridin)
4,5-Dimethylthiazol-2-azo-6′-(2′-brom-3′-hydroxypyridin)
6-Ethoxybenzthiazol-2-azo-6′-(2′-brom-3′-hydroxypyridin)
4′-Cyanopyrazol-3-azo-2′-(4′-methoxyphenol)
4,5-Dimethylthiazol-2-azo-2′-(5′-chlor-3′-hydroxypyridin).

Für die Herstellung der erfindungsgemäßen Verbindungen der genannten allgemeinen Formel wird zuerst in an sich bekannter Weise nach literaturbekannten Verfahren der Azofarbstoff mit den jeweils gewünschten Gruppen A und B hergestellt. Beispiele und Literatur für übliche Herstellungsverfahren finden sich z.B. in Ullmanns Enzyklopädie der Technischen Chemie, 4. Auflage, Verlag Chemie, Band 8, S. 244.

Chromogene Substrate für den Nachweis von Phosphatasen und Sulfatasen lassen sich durch Umsetzen der entsprechenden Azofarbstoffe mit geeigneten Säurehalogeniden herstellen. Üblicherweise wird für die Herstellung von Phosphatasesubstraten ein Azofarbstoff mit Phosphoroxychlorid umgesetzt, während für die Herstellung von Sulfatasesubstraten der Azofarbstoff mit Chlorsulfonsäure umgesetzt wird.

Für die Herstellung von Glykosiden werden die Azofarbstoffe nach ebenfalls bekannten Verfahren glykosiliert. Die Herstellung von ß-Galactosiden kann beispielsweise durch Umsetzung der entsprechenden Azofarbstoffe mit α-D-Acetobromgalactose und anschließender Entacetylierung hergestellt werden. Glykosilierungsverfahren sind z.B. beschrieben in Angewandte Chemie 98 (1986) Seiten 213 - 236 und darin zitierter Literatur. Beispiele für nach den genannten Verfahren erhältliche Glykoside sind z.B. α - und ß-D-Galactopyranoside, α - und ß-D-Glucopyranoside und sich daraus ableitende Oligosaccharid-Derivate mit 2-10, vorzugsweise 3-7 Monosaccharideinheiten.

Die erfindungsgemäßen chromogenen Substrate werden für den Nachweis verschiedener hydrolytischer Enzyme, beispielsweise Phosphatasen, Sulfatasen und Glykosidasen, verwendet. Für den Enzymnachweis wird das chromogene Substrat in einer Reagenzmischung zur Verfügung gestellt, die gegebenenfalls notwendige Puffersubstanzen, Stabilisatoren, Aktivatoren, Lösungsvermittler, Hilfsenzyme oder weitere Hilfschemikalien enthalten kann. Die verschiedenen Einzelchemikalien können bei hinreichender Stabilität und chemischer Verträglichkeit in einer Lösung nebeneinander vorliegen, sie können aber auch erst kurz vor der Nachweisreaktion miteinander vermischt werden. Der eigentliche Nachweis des hydrolytisch wirksamen Enzyms findet dann durch Messung der Extinktion des durch die enzymkatalysierte Hydrolyse freigesetzten Azofarbstoffes aus der entsprechenden Azofarbstoffverbindung nach Zusammenbringen der Reagenzmischung mit dem nachzuweisenden hydrolysierenden Enzym bzw. der zu testenden biologischen Probe statt. Bevorzugt ist dabei eine Reaktion in Lösung, die gegebenenfalls direkt in einer Küvette durchgeführt werden kann und sofort durch anschließende, durchlichtphotometrische Signalermittlung ausgewertet werden kann. Ebenso bevorzugt ist die Applikation der erfindungsgemäßen chromogenen Substrate auf fibröse oder filmartige Reagenzienträger, die nach Durchführen der Reaktion eine reflexionsphotometrische Signalermittlung gestatten. In beiden Fällen ist bei Verwendung der erfindungsgemäßen chromogenen Substrate eine visuelle Auswertung ebenso möglich.

Die erfindungsgemäßen chromogenen Substrate können in verschiedener Form zur Verfügung gestellt werden. Bevorzugt sind dabei Ausführungsformen, die bereits eine Kombination der erfindungsgemäßen chromogenen Substrate mit testnotwendigen Zusatzreagenzien enthalten. Beispiele dafür sind Lösungen, Reagenzientabletten, Pulvergemische oder Lyophilisate, wenn die Nachweisreaktion anschließend in Lösung durchgeführt werden soll. Alternativ können die chromogenen Substrate in Verbindung mit den testnotwendigen Zusatzreagenzien ebenfalls auf saugfähige Träger aufgezogen oder in hydrophile und wasseraufnehmende Filme eingearbeitet werden.

Die Erfindung wird durch die folgende Figur und die Beispiele erläutert.

### Fig.1

Die Figur veranschaulicht die Veränderung des Absorptionsmaximums eines chromogenen Substrates, nämlich Thiazol-2-azo-2′-pyridin-3′-ol (Tap).

Dabei entspricht der Peak bei 376 nm (Spektrum 1) dem Azofarbstoff-Galactosid, das Absorptionsmaximum bei 468 nm (Spektrum 2) dem freien Chromogen, das Absorptionsmaximum bei 526 nm (Spektrum 3) schließlich dem Kupferkomplex des freien Chromogens. Der Vergleich der Spektren für das Galactosid und den Kupferkomplex zeigt deutlich, daß sich die entsprechenden Absorptionskurven nicht überlagern.

### Beispiel 1

### Herstellung von Thiazol-2-azo-2′-pyridin-3′-ol (Tap)

### Diazotierung:

5 g (50 mMol) 2-Aminothiazol werden in 75 ml halbkonzentrierter Salzsäure gelöst. Innerhalb von 30 min werden bei 0 bis 4°C unter Rühren 30 ml Natriumnitrit-Lösung (117 g/l = 1,7 mol/l) zugetropft. Nach beendeter Zugabe wird die Reaktionsmischung 30 min. gerührt.

### Kupplung:

In 500 ml 0,1 M NaOH werden 4,3 g (45 mMol) 3-Hydroxypyridin gelöst und mit Eiswürfeln auf 0 bis 5°C gekühlt. Diese Temperatur wird bis zur Beendigung der Kupplung gehalten.

Unter pH-Kontrolle wird das ebenfalls gekühlte Diazoniumsalz in die Pyridinollösung getropft. Durch Zutropfen von 10 M Natronlauge wird der pH-Wert während der Kupplung zwischen pH 7 und pH 10 gehalten.

### Isolierung:

Nach beendeter Kupplung wird die Lösung mit konzentrierter Salzsäure auf pH 3 eingestellt, der entstehende Niederschlag abgesaugt und im Exsikkator über Phosphorpentoxid im Vakuum getrocknet.

### Reinigung:

Das trockene Rohprodukt wird nach Zerkleinerung im Soxhlet mit 1 l Toluol extrahiert. Das im Kolben auskristallisierte Tap wird abgesaugt und anschließend gegebenenfalls mehrmals aus Toluol und/oder Wasser umkristallisiert. Bei langsamer Abkühlung entstehen rotbraune Nadeln.

Die Ausbeute nach der Reinigung betrug ca. 1,5 g Tap.

### Charakterisierung:

Das erhaltene Produkt wurde durch Dünnschichtchromatographie, Schmelzpunktbestimmung und NMR-Spektroskopie charakterisiert.
- A:: Dünnschichtchromatographie: Laufmittel Chloroform/Methanol 90/10 auf Kieselgel-Platten der Fa. Merck; Rf-Wert von 0,38.
- B:: Die Schmelzpunktbestimmung ergab einen Schmelzpunkt Fp > 170°C unter Zersetzung
- C:: NMR-Spektroskopie: Das NMR-Spektrum wies folgende Peaks auf:

1. Duplett bei 8,4 ppm koppelt mit 5.
2. Duplett bei 8,2 ppm koppelt mit 4.
3. Duplett bei 8,1/8,2 ppm koppelt mit 5.
4. Duplett bei 7,9 ppm koppelt mit 2.
5. Quadruplett bei 7,5/7,6 ppm koppelt mit 1. und 3.
6. mehrere Einzelpeaks von 1,5 bis 5,5 ppm.

Jede Position stellt gemäß Integrationskurve ein Wasserstoffatom dar.

**Tabelle 1**

| Hydroxyfarbstoffe, hergestellt analog den in den Beispielen 1 und 2 beschriebenen Verfahren | | | |
|---|---|---|---|
| Azofarbstoff | Extinktionsmax. (nm)/pH | | εₘₒₗ / λₘₐₓ Salz (nm) |
| | Säure | Salz | |
| Thiazol-2-azo-2'-pyridin-3'-ol | 376/4 | 468/ 6 | 22.000 / 468 |
| 4,5-Dimethylthiazol-2-azo-2'pyridin-3'-ol | 425/6 | 480/ 7 | 41.000 / 480 |
| 6-Ethoxybenzthiazol-2-azo-2'-pyridin-3'-ol | 440/5 | 500/ 7 | 17.000 / 500 |
| Benzthiazol-2-azo-2'-pyridin-3'-ol | 390/5 | 490/ 7 | 24.000 / 490 |
| 5-Bromthiazol-2-azo-2'-pyridin-3'-ol | 410/5 | 490/ 7 | 21.000 / 490 |
| Thiazol-2-azo-6'-(2'-brom-3'-hydroxypyridin) | 400/4 | 470/ 6 | 25.000 / 470 |
| 4,5-Dimethylthiazol-2-azo-6'-(2'-brom-3' -hydroxypyridin) | 440/4 | 490/ 6 | 32.000 / 490 |
| 6-Ethoxybenzthiazol-2-azo-6'-(2'-brom-3'-hydroxypyridin | 400/4 | 500/6 | 32.000 / 500 |
| 4,5-Dimethylthiazol-2-azo-2'-(5'-chlor-3'-hydroxypyridin | 420/./. | 490/./. | 36.600 / 490 |

Neben den hier aufgeführten Beispielen wurden außerdem noch weitere hydroxygruppenhaltige Derivate der Substanzklassen Naphtole und Hydroxyindole als Kupplungskomponenten eingesetzt. Die zur Diazotierung eingesetzten Aminkomponenten sind folgender Aufzählung zu entnehmen: Aminothiazole (u.a. substituiert mit Alkyl-, Halogen-, Nitro-Phenylgruppen), Aminobenzthiazolderivate, Aminopyridinderivate, Aminothiadiazolderivate, Aminoisothiazolderivate, Aminooxazol- und Aminoisoxazolderivate, Anilinderivate, Aminonsphthalinderivate.

### Beispiel 2 :

Komplexierung von Hydroxyazofarbstoffen.

Das Komplexierungsverhalten verschiedener Verbindungen wurde wie folgt untersucht:
In eine Küvette wurden in der angegebenen Reihenfolge einpipettiert und sofort verrührt:
2,960 ml 0,1 M Phosphatpuffer pH 7, 30 ul einer 10 »M Lösung des Azofarbstoffes in Methanol und 10 ul einer 0,1 M wäßrigen Lösung des komplex-bildenden Metallions.

Qualitative Aussagen wurden wie folgt erhalten:
Ein mit 0,1 M Phosphatpufferlösung pH 7 vorimpägniertes und getrocknetes Indikatorpapier wurde mit einer methanolischen Lösung der untersuchten Verbindung versetzt. Nach dem Trocknen wurde eine 0,3 mM Lösung des komplex-bildenden Metallions aufgetropft.

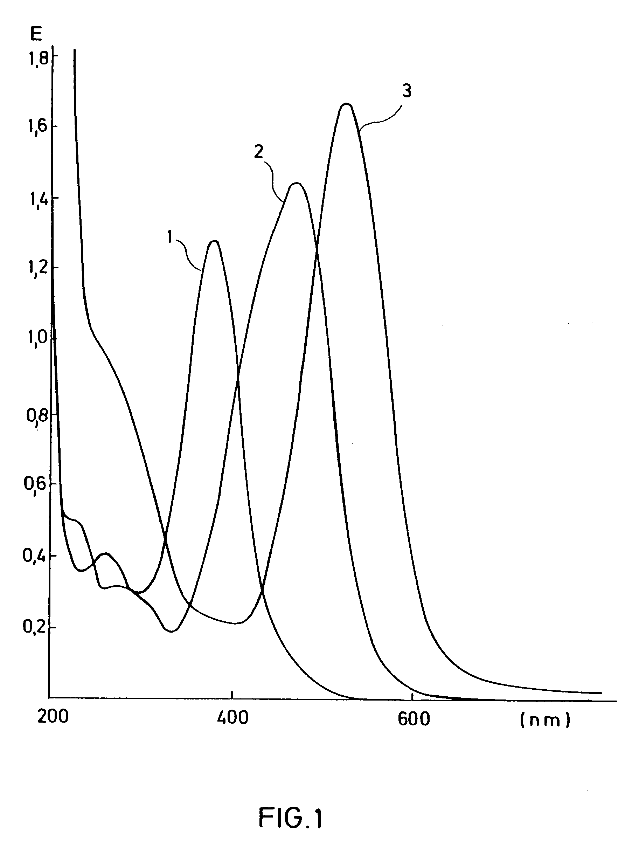

Ein Beispiel für die Farbverschiebung durch Komplexierung zeigt Fig. 1.

Weitere Beispiele sind in Tabelle 2 zusammengestellt.

**Tabelle 2**

| Komplexierung von Hydroxyazofarbstoffen | | |
|---|---|---|
| Azofarbstoff | Komplexatom | εₘₒₗ / λₘₐₓ |
| Thiazol-2-azo-2'-pyridin-3-ol | Cu | 27.550 / 526 |
| | Co | 25.000 / 490 |
| | Ni | 32.000 / 500 |
| 4,5-Dimethylthiazol-2-azo-2'-pyridin-3'-ol | Cu | 46.000 / 538 |
| Benzthiazol-2-azo-2-pyridin-3'-ol | Cu | 25.000 / 540 |
| 5-Chlorthiazol-2-azo-2'-pyridin-3'-ol | Cu | 16.000 / 540 |
| 5-Bromthiazol-2-azo-2'-pyridin-3'-ol | Cu | 29.000 / 550 |
| 4,5-Dimethylthiazol-2-azo-2'-(5'-chlor-3'-hydroxypyridin) | Cu | 38.300 / 540 |

### Beispiel 3 :

### Glykosylierungsreaktionen

### A. Galactosylierung von Thiazol-2-azo-2'-pyridin-3' -ol (Tap)

In einer trockenen Apparatur werden 2,06 g (0,01 Mol) Tap, 4,1 g (0,01 Mol) Acetobromgalactose, 1,15 g (5 mMol) Ag₂O und 1,45 g (0,01 Mol) Calziumsulfat · 1/2 H₂O in 100 ml getrocknetem Toluol unter Rückfluß erhitzt.

Die Reaktion erfolgt unter Ausschluß von Licht und Wasser (Calziumchloridtrockenrohr auf dem Kühler) unter Rühren.

Nach 2 Stunden Reaktionszeit wird die Umsetzung dünnschichtchromatographisch kontrolliert. Das entstandene Tap-acetogalactosid erscheint als gelber Fleck über dem Tap-Fleck. Laufmittel Chloroform/Ethylacetat 4/1 bzw. Chloroform/Aceton 4/1.

Bei nicht ausreichender Umsetzung werden nochmals Acetobromgalactose und Ag₂O zugesetzt und die Mischung wiederum unter Rückfluß erhitzt.

Nach erfolgter Umsetzung wird die Ansatzlösung filtriert, das Filter mit Toluol gewaschen, Waschtoluol und Mutterlauge vereint und am Rotationsverdampfer bei max. 40°C Badtemperatur bei ca. 70 millibar eingeengt.

Der Rückstand wird im Vakuumtrockenschrank bei Raumtemperatur und 200 mbar getrocknet. Das verunreinigte Produkt wird durch Säulenchromatographie gereinigt (Trennmittel Kieselgel 40, 0,06-0,2 mm, Laufmittel Chloroform/Ethylacetat 4/1). Das gereinigte Tap-tetraacetylgalactosid wird getrocknet und seine Reinheit dünnschichtchromatographisch überprüft.

### B. Entacetylierung des entstandenen Acetylglykosids

Die Entacetylierung wird in wasserfreiem Methanol mit Natriummethylat durchgeführt. Dazu wird das Acetylglykosid (2 mg/ml) in Methanol gelöst und wenig Natriummethylat zugesetzt (5-10 »l einer 30%igen methanolischen Lösung). Der Verlauf der Entacetylierung wird mittels Dünnschichtchromatographie überwacht.

Laufmittel: Chloroform/Ethylacetat 4/1 und Chloroform/Methanol 3/1.

Nach Vollendung der Reaktion wird die Lösung mit Ionenaustauscher Dowex 50 x 8 neutral bis schwach sauer eingestellt.

Anschließend wird die Lösung eingeengt und getrocknet. Die Ausbeute an Tap-β-galactosid betrug 180 mg.

### C.Glucosilierung von 3,5-Dimethylthiazol-2-azo-2'-pyridin-3'-ol (Dimetap)

2,63 g (0,01 Mol) Brigl's Anhydrid und 2,36 g (0,01 Mol) Dimetap werden in 100 ml Toluol unter Rückfluß und Rühren sowie Ausschluß von Wasser erhitzt.

Reaktionszeit: 24 - 48 h.

Die Umsetzung wird dünnschichtchromatographisch überprüft.

Laufmittel: Chloroform/Ethylacetat 4/1.

Die Entacetylierung erfolgt wie unter B. beschrieben.

Die Ausbeute betrug 200 mg Dimetap-β-glucosid.

### Beispiel 4

Umsetzung von chromogenen Galactosiden mit β-Galactosidasen
Thiazol-2'-azo-2'-pyridin-3'-β-galactosid (Tap-Gal) wurde mit β-Galactosidasen aus E.coli (EC 3.2.1.22, pH-Optimum ca. 7,2) und Asp.orycae (EC 3.2.1.23, pH-Optimum ca. 4,5), Calbiochem GmbH, abgebaut. Der Abbau wurde visuell und photometrisch verfolgt. Zum Vergleich wurden parallel dazu, die Abbaugeschwindigkeiten der bekannten chromogenen β-Galactosidasesubstrate Resorufin - β-galactosid (Res-Gal) und Chlorphenolrot-β-galactosid (CPR-Gal) ermittelt.

### Photometrisches Verfahren

In einer Küvette mit 1 cm Lichtweg wird 1 ml einer 0,1 M Pufferlösung (Phosphatpuffer pH 7 bzw. Acetatpuffer pH 4,5) mit 0,1 ml der jeweiligen Substratlösung (2,5 mMol/l bzw. 10 mMol/l vermischt und die Abbaureaktion durch Zugabe von 10 »l einer β-Galactosidaselösung (840 U/l) gestartet. Die Abbaureaktion wird ca. 10 min verfolgt. Bei pH 4,5 enthält die Tap-Lösung noch 0,33 mMol/l Kupfernitrat, die beiden anderen Substrate werden nach Umpufferung auf pH 7 vermessen.

**Tabelle 3**

| Ergebnisse der photometrischen Untersuchungen | | | | |
|---|---|---|---|---|
| chromgenes Substrat | Abbaugeschwindigkeit (»mol/min) bei verschiedene Substratkonzentrationen | | | |
| | 0,25 (mMol/l) | | 1,0 (mMol/l) | |
| | E.coli | Asp.orycae | E.coli | Asp.orycae |
| Tap-Gal | 0,35 | 0,7 | 0,6 | 1,5 |
| Res-Gal | 0,4 | 1,1 | 0,2* | ./.* |
| CPR-Gal | 0,27 | 0,15 | 0,57 | 0,49 |

| | | | | |
|---|---|---|---|---|
| * Wegen der schlechten Löslichkeit dieses Substrates in Wasser muß ein erheblicher Anteil an Methanol/Dimethylformamid zugefügt werden, welcher dann die Enzyme inhibiert. | | | | |

### Visuelles Verfahren

Indikatorpapier wurde mit einer 0,1 M Pufferlösung (Phosphatpuffer pH 6, pH 7 oder pH 7,8 bzw. Citratpuffer pH 4,5) vorimprägniert, getrocknet und mit einer methanolischen Lösung von 1 g/l des jeweiligen Substrates nachimprägniert und getrocknet. Die für Tap-Gal vorgesehenen Papiere enthielten in den Puffern pH 4,5, pH 6 und pH 7 zusätzlich 0,3 mMol/l Kupfernitrat.

Die so erhaltenen Galactosidase-Testpapiere wurden in wäßrige Lösungen fallender Galactosidasekonzentration getaucht und die Farbentwicklung gegen einen Nullwert verglichen. Tabelle 4 enthält die noch nachweisbaren Minimalkonzentrationen an Enzym, wobei für Res-Gal und CPR-Gal vor dem Ablesen auf pH 7 umgepuffert wurde, sofern vorher bei pH 4,5 oder pH 6 gearbeitet wurde.

**Tabelle 4**

| Maximale Sensitivität von Chlorphenolrotgalactosid (CPR), Resorufingalactosid (Res) und Thiazolylazopyridinolgalactosid (Tap) im Eintauchtest in U/l | | | | | | | |
|---|---|---|---|---|---|---|---|
| Enzym | Puffer | pH | Testdauer (min) | Substrate | | | Tap + Cu⁺⁺ |
| | | | | CPR | Res | Tap | |
| E. coli Galactosidase | Phosphat | 7,8 | 1 | 840 | 840 | 840 | ./. |
| | | | 4 | 210 | 210 | 210 | ./. |
| A. orycae Galactosidase | Citrat | 4,5 | 1 | ./. | 240 | ./. | 240 |
| | | | 4 | 500 | 60 | | 60 |
| | Phosphat | 6 | 1 | 2.400 | 240-480 | ./. | 240-480 |
| | | | 4 | 800 | 120 | | 120 |
| | Phospat | 7 | 1 | ./. | 960 | ./. | 960 |
| | | | 4 | 2.000 | 240 | | 240 |

### Beispiel 5 :

Umsetzung von 4,5-Dimethylthiazol-2-azo-2'-pyridin-3'-β-D-glucopyranosid (Dimetap-Gluc) mit β-Glucosidase (EC 3.2.1.21).

In einer Küvette mit 1 cm Lichtweg werden 2,9 ml 0,1 M Phosphatpuffer pH 7 mit 0,1 ml einer 1,0 g/l enthaltenden wäßrigen Lösung von Dimetap-Gluc und 0,1 ml einer 10 mM Kupfernitratlösung vermischt und die Reaktion durch Zugabe von 0,1 ml einer β-Glucosidaselösung (Calbiochem GmbH) gestartet. Nach 15-minütiger Reaktionszeit ist das Substrat vollständig in freies Chromogen und Glucose gespalten (photometrische Kontrolle).

**Tabelle 5**

| | Testkonz. | Testakt. der alkalischen Phosphatase | Hg 578 mm bzw. Hg 623 mm |
|---|---|---|---|
| | »Mol/l | U/l | mE/min |
| Nitrotaphphosphat | 19 | 199 | 89 |
| | 65 | 199 | 265 |
| Indoxylphosphat | 4.900 | 95 | 99 |
| Testbedingungen: Glycinpuffer 0,1 Mol/l pH 10,5 0,1 mMol/l Zn⁺⁺ 1 mMol/l Mg⁺⁺ | | | |

### Beispiel 6 :

### Herstellung und Umsetzung von 5-Nitrothiazol-2-azo-4'-phenyl-sulfat (Nitrotaph-sulfat)

### Herstellung:

In 5 ml Pyridin wurden unter Rühren 0,84 ml (12,5 mMol) Chlorsulfonsäure langsam zugetropft; dabei wurde mit einer Eis-Kochsalzmischung gekühlt. In die entstandene Suspension wurden 500 mg NitroTaph eingetragen und die Mischung bei 40°C im Waserbad gerührt. Nach 25 h wurde das Reaktionsgemisch mit 20 ml Wasser versetzt, mit 2 M Kalilauge neutralisiert und am Rotationsverdampfer zur Trockne eingeengt.

### Reinigung:

150 mg Rohprodukt wurden in Wasser/Methanol-Gemisch gelöst und auf einer präparativen Kieselgelplatte (Fa. Merck) aufgetrennt.
Laufmittel: Essigester/Wasser/Methanol 100/25/30 (Rf. ca. 0,4).

### Umsetzung mit Arylsulfatase (E.C. 3.1.6.1):

Das Nitrotaph-sulfat wurde mit Methanol vom Kieselgel gelöst (Eluat). Ein Filtrierpapier wurde mit 0,1 M Acetatpuffer pH 6,2 getränkt und getrocknet. Darauf wurden verschiedene Verdünnungen des Eluats aufgetropft und mit Arylsulfatase (Fa. Boehringer Mannheim) inkubiert. Innerhalb 1 min erfolgte mit 80 U/l ein gut sichtbarer Farbumschlag von gelb nach grün.

## Patentansprüche

1. Chromogene Substrate zum Nachweis von hydrolytischen Enzymen dadurch gekennzeichnet, daß das Substrat eine Azofarbstoffverbindung der Formel I ist:
A -- N == N -- B (OR) (I)
worin
A einen zyklischen 5- und 6-gliedrigen, gegebenenfalls benzo-anelierten Rest mit bis zu 3 Heteroatomen aus der Gruppe N, S und O bedeutet, der gegebenenfalls durch Halogen-, Nitro-, Alkyl-, Alkoxy- und Sulfonatgruppen substituiert sein kann,
B Pyridinyl bedeutet, das gegebenenfalls durch Halogen-, Alkyl-, Alkoxy-, Dialkylamino- und Morpholinogruppen substituiert sein kann, und
R ein durch enzymatische Hydrolyse freisetzbarer Rest ist, ausgenommen ein Carbonylrest.

2. Chromogene Substrate nach Anspruch, 1 dadurch gekennzeichnet, daß R ein modifizierter oder unmodifizierter Zuckerrest oder ein Phosphatrest oder ein Sulfatrest ist.

3. Chromogene Substrate nach mindestens einem der Ansprüche 1 oder 2 dadurch gekennzeichnet, daß das Produkt der Hydrolyse des chromogenen Substrates zur Bildung von Komplexen mit Metallionen geeignet ist.

4. Chromogene Substrate nach mindestens einem der Ansprüche 1 bis 3 dadurch gekennzeichnet, daß sie Glykoside, Phosphorsäure- oder Sulfatester der folgenden Azofarbstoffe sind:
Thiazol-2-azo-2'-pyridin-3'-ol-4,5-Dimethylthiazol-2-azo-2'-pyridin-3'-ol
6-Ethoxybenzthiazol-2-azo-2'-pyridin-3'-ol
Benzthiazol-2-azo-2'-pyridin -3'-ol
5-Bromthiazol-2-azo-2'-pyridin-3'-ol
Thiazol-2-azo-6'-(2'-brom-3'-hydroxypyridin)
4,5-Dimethylthiazol-2-azo-6'-(2'-brom-3'-hydroxypyridin)
6-Ethoxybenzthiazol-2-azo-6'-(2'-brom-3'-hydroxypyridin)
4,5-Dimethylthiazol-2-azo-2'-(5'-Chlor-3'-hydroxypyridin).

5. Verfahren zum Herstellen von chromogenen Substraten nach mindestens einem der Ansprüche 1 bis 4 dadurch gekennzeichnet, daß ein Azofarbstoff der allgemeinen Formel II
A - N = N - B(OH) (II)
mit einem Säurehelogenid umgesetzt wird.

6. Verfahren zum Herstellen von chromogenen Substraten nach Anspruch 5 dadurch gekennzeichnet, daß das Säurehalogenid Phosporoxychlorid ist.

7. Verfahren zum Herstellen von chromogenen Substraten nach Anspruch 5 dadurch gekennzeichnet, daß das Säurehalogenid Chlorsulfonsäure ist.

8. Verfahren zum Herstellen von chromogenen Substraten nach mindestens einem der Ansprüche 1 bis 4 dadurch gekennzeichnet, daß ein Azofarbstoff der allgemeinen Formel
A - N = N - B(OH)
mit Acetobromgalaktose und anschließend mit Natriummethylat umgesetzt wird.

9. Verwendung eines chromogenen Substrates nach mindestens einem der Ansprüche 1 bis 4, wobei R ein Phosphatrest ist, zum Nachweis von Phosphatasen.

10. Verwendung eines chromogenen Substrates nach mindestens einem der Ansprüche 1 bis 4, wobei R ein Sulfatrest ist, zum Nachweis von Sulfatasen.

11. Verwendung eines chromogenen Substrates nach mindestens einem der Ansprüche 1 bis 4, wobei R ein modifizierter oder unmodifizierter Zuckerrest ist, zum Nachweis von Glykosidasen.

12. Reagenzmischung für den Nachweis hydrolysierender Enzyme, enthaltend ein oder mehrere chromogene Substrate nach mindestens einem der Ansprüche 1 bis 4 und gegebenenfalls mindestens eine der folgenden Substanzen:
Puffersubstanzen,
Aktivatoren,
Lösungsvermittler,
Hilfsenzyme,
weitere Hilfschemikalien.

13. Reagenzmischung nach Anspruch 12, wobei das chromogene Substrat gegebenenfalls in Verbindung mit testnotwendigen Zusatzreagenzien in Lösung, als Reagenzientablette, Als Pulvergemisch oder Lyophilisat zur Verfügung gestellt wird.

14. Reagenzmischung nach Anspruch 12, wobei das chromogene Substrat gegebenenfalls in Verbindung mit testnotwendigen Zusatzreagenzien auf saugfähige Träger aufgezogen oder in hydrophile Filme eingearbeitet ist.

## Claims

1. A chromogenic substrate for detection of a hydrolyzing enzyme, in which the substrate is an azo dye-stuff compound of the formula I:
A - N = N - B (OR) (I)
in which
A denotes a cyclic 5- or 6-membered, optionally benzo-fused radical with up to 3 hetero atoms from the group comprising N, S and O, which can optionally be substituted by halogen, nitro, alkyl, alkoxy or sulfonate groups;
B denotes pyridinyl which can optionally be substituted by halogen, alkyl, alkoxy, dialkylamino or morpholino groups and
R is a radical which can be liberated by enzymatic hydrolysis, excluding a carbonyl radical.

2. A chromogenic substrate as claimed in claim 1, wherein R is a modified or non-modified sugar radical or a phosphate radical or a sulfate radical.

3. A chromogenic substrate as claimed in at least one of claims 1 and 2, wherein the product of the hydrolysis of the chromogenic substrate is suitable for the formation of complexes with metal ions.

4. A chromogenic substrate as claimed in at least one of claims 1 to 3, which is a glycoside, phosphoric acid ester or sulfate ester of the following azo dyestuffs:
thiazole-2-azo-2'-pyridin-3'ol
4,5-dimethylthiazole-2-azo-2'-pyridin-3'-ol
6-ethoxybenzothiazole-2-azo-2'-pyridin-3'-ol
benzothiazole-2-azo-2'-pyridin-3'-ol
5-bromothiazole-2-azo-2'-pyridin-3'-ol
thiazole-2-azo-6'-(2'-bromo-3'-hydroxypyridine)
4,5-dimethylthiazole-2-azo-6'-(2'-bromo-3'-hydroxypyridine)
6-ethoxybenzothiazole-2-azo-6'-(2'-bromo-3'-hydroxypyridine)
4,5-dimethylthiazole-2-azo-2'-(5'-chloro-3'-hydroxypyridine).

5. A process for the preparation of a chromogenic substrate as claimed in at least one of claims 1 to 4, which comprises reacting an azo dyestuff of the formula
A - N = N - B(OH) (II)
with an acid halide.

6. The process for the preparation of a chromogenic substrate as claimed in claim 5, wherein the acid halide is phosphorus oxychloride.

7. The process for the preparation of a chromogenic substrate as claimed in claim 5, wherein the acid halide is chlorosulfonic acid.

8. The process for the preparation of a chromogenic substrate as claimed in at least one of claims 1 to 4, which comprises reacting an azo dyestuff of the formula
A - N = N - B(OH)
with acetobromogalactose and then with sodium methylate.

9. The use of a chromogenic substrate as claimed in at least one of claims 1 to 4, in which R is a phosphate radical, for the detection of a phosphatase.

10. The use of a chromogenic substrate as claimed in at least one of claims 1 to 4, in which R is a sulfate radical, for the detection of a sulfatase.

11. The use of a chromogenic substrate as claimed in at least one of claims 1 to 4, in which R is a modified or non- modified sugar radical, for the detection of a glycosidase.

12. A reagent mixture for the detection of a hydrolyzing enzyme, containing one or more chromogenic substrates as claimed in at least one of claims 1 to 4 and if appropriate at least one of the following substances:
buffer substances,
activators,
solubilizing agents,
auxiliary enzymes,
other auxiliary chemicals.

13. A reagent mixture as claimed in claim 12, in which the chromogenic substrate is made available in solution, as a reagent tablet, as a powder mixture or as a lyophilisate, if appropriate in combination with additional reagents needed for the test.

14. A reagent mixture as claimed in claim 12, in which the chromogenic substrate is absorbed onto an absorbent carrier or incorporated into a hydrophilic film, if appropriate in combination with additional reagents needed for the test.

## Revendications

1. Substrats chromogènes pour la détection d'enzymes hydrolytiques, caractérisés en ce que le substrat est un dérivé de colorant azoïque, de formule I
A-N=N-B(OR)
dans laquelle
A représente un radical cyclique à 5 ou 6 chaînons, comportant jusqu'à 3 hétéroatomes choisis parmi N, S, O, éventuellement soudé à un noyau benzénique et qui peut éventuellement être substitué par des halogènes ou par des groupes nitro, alkyle, alcoxy ou sulfonate;
B représente un radical pyridinyle qui peut éventuellement être substitué par des halogènes ou par des groupes alkyle, alcoxy, dialkyamino ou morpholino, et
R est un radical séparable par hydrolyse enzymatique, a l'exception d'un radical carbonyle.

2. Substrats chromogènes selon la revendication 1, caractérisés en ce que R est un radical glucidique modifié ou non modifié ou un radical phosphate ou un radical sulfate.

3. Substrats chromogènes selon au moins l'une des revendications 1 et 2, caractérisés en ce que le produit de l'hydrolyse du substrat chromogène est approprié à la formation de complexes avec des ions métalliques.

4. Substrats chromogènes selon au moins l'une des revendications 1 à 3, caractérisés en ce qu'ils consistent en des glycosides, esters phosphoriques ou sulfuriques des colorants azoïques suivants:
thiazol-6-azo-2'-pyridine-3'-ol,
4,5-diméthylthiazol-2-azo-2'-pyridine-3'-ol,
6-éthoxybenzothiazol-2-azo-2'-pyridine-3'-ol,
benzothiazol-2-azo-2'-pyridine-3'-ol,
5-bromothiazol-2-azo-2'-pyridine-3'-ol,
thiazol-2-azo-6'-(2'-bromo-3'-hydroxypyridine),
4,5-diméthylthiazol-2-azo-6'-(2'-bromo-3'-hydroxypyridine),
6-éthoxybenzothiazol-2-azo-6'-(2'-bromo-3'-hydroxypyridine),
4,5-diméthylthiazol-2-azo-2'-(5'-chloro-3'-hydroxypyridine).

5. Procédé pour la préparation de substrats chromogènes selon au moins l'une des revendications 1 à 4, caractérisé en ce que l'on fait réagir avec un halogénure d'acide un colorant azoïque de formule générale II
A-N=N-B(OH) (II)

6. Procédé pour la préparation de substrats chromogènes selon la revendication 5, caractérisé en ce que l'halogénure d'acide est l'oxychlorure de phosphore.

7. Procédé pour la préparation de substrats chromogènes selon la revendication 5, caractérisé en ce que l'halogénure d'acide est l'acide chlorosulfonique.

8. Procédé pour la préparation de substrats chromogènes selon au moins l'une des revendications 1 à 4, caractérisé en ce que l'on fait réagir un colorant azoïque de formule générale
A-N=N-B(OH)
avec de l'acétobromogalactose et ensuite avec du méthylate de sodium.

9. Utilisation d'un substrat chromogène selon au moins l'une des revendications 1 à 4, dans lequel R est un reste phosphate, pour la détection de phosphatases.

10. Utilisation d'un substrat chromogène selon au moins l'une des revendications 1 à 4, dans lequel R est un reste sulfate, pour la détection de sulfatases.

11. Utilisation d'un substrat chromogène selon au moins l'une des revendications 1 à 4, R étant un radical glucidique modifié ou non modifié, pour la détection de glucosidases.

12. Mélange de réactifs pour la détermination d'enzymes hydrolytiques, contenant un ou plusieurs substrats chromogènes selon au moins l'une des revendications 1 à 4, et éventuellement au moins l'une des substances suivantes:
substances tampons
activateurs
tiers-solvants
enzymes auxiliaires
autres produits chimiques auxiliaires.

13. Mélange de réactifs selon la revendication 12, dans lequel le substrat chromogène est présenté, éventuellement en association avec des réactifs supplémentaires requis pour l'essai, en **solution**, sous forme de comprimés de réactifs, sous forme d'un mélange pulvérulent ou d'un lyophilisat.

14. Mélange de réactifs selon la revendication 12, dans lequel le substrat chromogène, éventuellement en association avec des réactifs supplémentaires requis pour l'essai, est appliqué sur des supports absorbants ou incorporés dans des pellicules hydrophiles.
